# EUROPEAN PATENT APPLICATION

(11) **EP 2 261 214 A1**
(43) Date of publication of application: **15.12.2010**
(21) Application number: 09162630.9
(22) Date of filing: 12.06.2009
(51) Int. Cl.: C07D 215/38, C07D 401/12, A61K 31/33, A61P 43/00

(54) **Compounds useful to treat premature aging and in particular progeria**

(71) Applicant: Splicos Sas, 39293 Montpellier (FR); CNRS (Centre National de La Recherche Scientifique), 75794 Paris Cedex 16 (FR); Institut Curie, 75248 Paris Cedex 05 (FR); Université Montpellier 2, 34095 Montpellier Cedex 5 (FR)
(72) Inventor: Mahuteau, Florence, 78470, SAINT REMY-LES-CHEVREUSE (FR); Tazi, Jamal, 34380, CLAPIERS (FR); Najman, Romain, 94240, L'HAY-LES-ROSES (FR)
(74) Representative: Le Coupanec, Pascale A.M.P.

(57) **Abstract**

The present invention relates to a compound of formula (I): 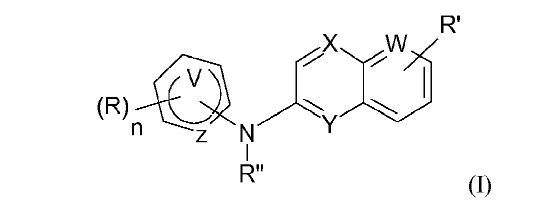 wherein: 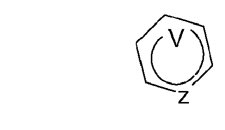 means an aromatic ring wherein V is C or N and when V is N, V is in ortho, meta or para of Z, i.e. forms respectively a pyridazine, a pyrimidine or a pyrazine group,
R independently represents a hydrogen atom, a halogen atom or a group chosen among a (C₁-C₃)alkyl group, a -CN group, a -COOH group, a (C₁-C₃)fluoroalkyl group, a (C₁-C₃)fluoroalkoxy group, a -NO₂ group, a -NR₁R₂ group and a (C₁-C₃)alkoxy group,
R₁ and R₂ are independently a hydrogen atom or a (C₁-C₃)alkyl group,
n is 1 or 2,
R' is a hydrogen atom or a group chosen among a (C₁-C₃)alkyl group, a halogen atom, a hydroxy group, a -COOH group and a -CN group,
R" is a hydrogen atom or a (C₁-C₄)alkyl group,
Z is N or C,
Y is N or C,
X is N or C,
W is N or C,

and wherein at most two of the groups Z, Y, X and W are N,
or anyone of its pharmaceutically acceptable salt,
for use as an agent for preventing, inhibiting or treating pathological or nonpathological conditions linked with premature aging.

## Description

### FIELD OF THE INVENTION

The present invention is generally dedicated to the use of compounds for the manufacture of compositions useful to treat diseases related to premature aging. The compounds and compositions containing them and compositions according to the invention may in particular be used to inhibit, prevent and/or treat Progeria.

### BACKGROUND OF THE INVENTION

The present invention focuses on premature aging. Said premature aging may be encountered in patients suffering from various diseases and in particular from the Hutchinson-Gilford progeria syndrome (HGPS) and from the HIV infection.

Hutchinson-Gilford progeria syndrome (HGPS) is a rare genetic disorder phenotypically characterized by many features of premature aging. It is clinically characterized by postnatal growth retardation, midface hypoplasia, micrognathia, premature atherosclerosis, absence of subcutaneous fat, alopecia and generalized osteodysplasia (Khalifa, 1989 - Hutchinson-Gilford progeria syndrome: report of a Libyan family and evidence of autosomal recessive inheritance. Clin. Genet. 35, 125-132.). At birth, the appearance of patients is generally normal, but by 1 year of age patients show severe growth retardation, balding and sclerodermatous skin changes. They average about 1m in height and usually weigh less than 15 kg even as teenagers. The age at death ranges from 7 to 28 years, with a median of 13.4 years. Over 80% of deaths are due to heart attacks or congestive heart failure.

Premature aging syndrome has been observed in patients suffering from HIV infections. One mechanical pathway underlying said premature aging could be associated, as for the HGPS and as exposed beneath, with an aberrant splicing of the nuclear lamin A gene. Indeed it has recently been hypothesized that protease inhibitors against HIValso block the transformation of prelamin A into lamin A as it turned out in HGPS.

Most of the patients suffering from premature aging carry a heterozygous silent mutation that activates the use of a cryptic 5' splice site in exon 11 of LMNA pre-mRNA. This aberrant splicing event leads to the production of a truncated protein (progerin) with a dominant negative effect which is responsible for the observed phenotype (De Sandre-Giovannoli et al., 2003 - Lamin A truncation in Hutchinson-Gilford progeria. Science 300, 2055 / Pendas et al., 2002a - Defective prelamin A processing and muscular and adipocyte alterations in Zmpste24 metalloproteinase-deficient mice. Nat. Genet. 31, 94-99.).

Most of the premature aging syndromes in particular associated withHutchinson-Gilford progeria and HIV infection are due to a recurrent, de novo point mutation in *LMNA* exon 11: c.1824C>T. This mutation is localized in the part of the gene specifically encoding lamin A (De Sandre-Giovannoli *et al.,* 2003 / De Sandre-Giovannoli and Levy, 2006 - Altered splicing in prelamin A-associated premature aging phenotypes. Prog. Mol. Subcell. Biol. 44, 199-232). Its predicted effect is a silent amino acid change at codon 608 (p.G608G). In fact, this sequence variation is not silent as it occurs in a probable exon splicing enhancer. As a result, a cryptic splice site is activated in transcripts issued from the mutated allele, which is located 5 nucleotides upstream of the mutation.

So far, therapeutic approaches have been mainly focused on progerin which is attached to a lipid anchor (a farnesyl lipid anchor). This lipid anchor is attached to progerin by a specific cellular enzyme, protein farnesyltransferase. Experiments in mouse models suggest that farnesyltransferase inhibitors (FTIs) may have beneficial effects in humans with progeria (Fong et al., 2006 - A protein farnesyltransferase inhibitor ameliorates disease in a mouse model of progeria. Science 311, 1621-1623). More recently, Nicolas Levy's team has used a combination of a statin and an aminobisphosphonate to prevent the fixation of the fatty acid to the progerin, and thus reduce its toxicity (Varela et al., 2008 - Combined treatment with statins and aminobisphosphonates extends longevity in a mouse model of human premature aging. Nat. Med. 14, 767-772.).

In WO2006/081444 has been reported a method for reducing at least one cellular defect in a cell from a subject susceptible to a disease or condition characterized by farnesylation on an abnormally farnesylated form of a lamin, comprising administering to the cell a therapeutically effective dose of farnesylstransferase inhibitor.

It has been recently reported in WO2008/003864 the use of a hydroxymethylglutaryl-coenzyme A (HMG-CoA) reductase inhibtor and a farnesyl-pyrophosphate synthase inhibitor, or one of their associated physiologically acceptable salts, in the preparation of a composition, for use in the treatment of human or animal, pathological or nonpathological situations related to the accumulation and/or the persistence of prenylated proteins in cells, such as during progeria, restrictive dermopathy or physiological aging.

In WO 2008/115870 substituted quinoline are described, which are useful for treating cancer.

In US 2008/0161353 other substituted quinoline are disclosed as agents to treat neurological conditions.

### SUMMARY OF THE INVENTION

It has now been found that derivatives of formula (I) as defined in formula (I) hereinafter are able to interfere with the usage of the cryptic splice and demonstrate efficient inhibition of aberrant splicing leading to progerin production as illustrated in the experimental data herein after and, on the basis of such activity, the compounds are useful in the treatment of premature aging and in particular of progeria.

The present invention therefore relates to compounds of formula (I) as defined below for use as agents for preventing, inhibiting or treating pathological or nonpathological conditions linked with premature aging.

The present invention moreover relates to a method of preventing, inhibiting or treating pathological or nonpathological conditions linked with premature aging, which comprises at least one step consisting in administering to a patient suffering there from an effective amount of a compound as defined in formula (I) below or one of its pharmaceutically acceptable salts.

The present invention further relates to some particular derivatives as such, as defined below.

The present invention also provides pharmaceutical compositions comprising at least one of said particular compounds.

### DETAILED DESCRIPTION OF THE INVENTION

The present invention is based on a novel approach based on the inhibition of aberrant splicing leading to progerin production.

The truncated Lamin A protein lacking the last 150 base pairs of exon 11 also called "progerin", acting as a dominant negative mutant, is predicted to be responsible for the characteristic manifestations seen in HGPS patients. Given that similar alteration of lamin A/C splicing was observed in aged individuals, it is proposed here that therapeutic molecules that interfere with the usage of the cryptic splice site will prevent side effects associated with accumulation of progerin during physiological aging. In other words, the compounds according to the present invention prevent usage of the cryptic 5' splice site in exon 11 of LMNA, allowing overcoming deleterious effect associated with progerin.

According to a first aspect, a subject-matter of the present invention relates to a compound of formula (I) 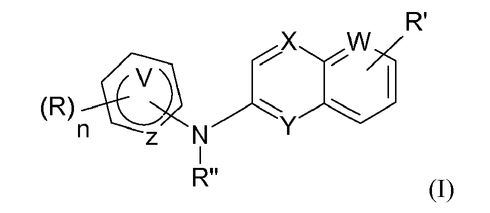 wherein: 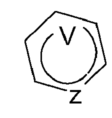
means an aromatic ring wherein V is C or N and when V is N, V is in ortho, meta or para of Z, i.e. forms respectively a pyridazine, a pyrimidine or a pyrazine group,
R independently represents a hydrogen atom, a halogen atom or a group chosen among a (C₁-C₃)alkyl group, a -CN group, a -COOH group, a (C₁-C₃)fluoroalkyl group, a (C₁-C₃)fluoroalkoxy group, a -NO₂ group, a -NR₁R₂ group and a (C₁-C₃)alkoxy group,
R₁ and R₂ are independently a hydrogen atom or a (C₁-C₃)alkyl group,
n is 1 or 2,
R' is a hydrogen atom or a group chosen among a (C₁-C₃)alkyl group, a halogen atom, a hydroxy group, a -COOH group and a -CN group,
R" is a hydrogen atom or a (C₁-C₄)alkyl group,
Z is N or C,
Y is N or C,
X is N or C,
W is N or C,
and wherein at most two of the groups Z, Y, X and W are N,
or anyone of its pharmaceutically acceptable salt,
for use as an agent for preventing, inhibiting or treating pathological or nonpathological conditions linked with premature aging.

According to one aspect, the present invention relates to a compound of formula (I) as defined above, wherein V is N, for use as an agent for preventing, inhibiting or treating pathological or nonpathological conditions linked with premature aging.

According to one aspect, the present invention relates to a compound of formula (I) as defined above, wherein Z is N, V is C, Y is N, X is C and W is C, for use as an agent for preventing, inhibiting or treating pathological or nonpathological conditions linked with premature aging.

According to another aspect, the present invention relates to a compound of formula (I) as defined above, wherein Z is C, V is C, Y is N, X is C and W is C, for use as an agent for preventing, inhibiting or treating pathological or nonpathological conditions linked with premature aging.

According to another aspect, the present invention relates to a compound of formula (I) as defined above, wherein Z is N, V is C, Y is C, X is N and W is C, for use as an agent for preventing, inhibiting or treating pathological or nonpathological conditions linked with premature aging.

According to another aspect, the present invention relates to a compound of formula (I) as defined above, wherein Z is N, V is C, Y is C, X is C and W is N, for use as an agent for preventing, inhibiting or treating pathological or nonpathological conditions linked with premature aging.

The compounds of the invention may exist in the form of free bases or of addition salts with pharmaceutically acceptable acids.

Suitable physiologically acceptable acid addition salts of compounds of formula (I) include hydrochloride, hydrobromide, tartrate, fumarate, citrate, trifluoroacetate, ascorbate, and malate.

The compounds of formula (I) and or salts thereof may form solvates (e.g. hydrates) and the invention includes all such solvates.

In the context of the present invention, the term:
- "halogen" is understood to mean chlorine, fluorine, bromine, or iodine, and in particular denotes chlorine, fluorine or bromine,
- "(C₁-C₃)alkyl" as used herein respectively refers to C₁-C₃ normal, secondary or tertiary saturated hydrocarbon. Examples are, but are not limited to, methyl, ethyl, 1-propyl, 2-propyl,
- "fluoroalkyl group" and "fluoroalkoxy group" refers respectively to alkyl group and alkoxy group as above-defined, said groups being substituted by at least one fluorine atom. Examples are perfluoroalkyl groups, such as trifluoromethyl or perfluoropropyl, and
- "patient" may extend to humans or mammals, such as cats or dogs.

In one particular variant, the present invention is directed to a compound of formula (I) wherein:
Z is N or C, Y is N or C, X is N or C and W is C,
n is equal to 1,
R is a hydrogen atom, a -COOH group, a (C₁-C₃)alkyl group or a (C₁-C₃)fluoroalkoxy group,
R' is a hydrogen atom,
R" is a hydrogen atom, and
wherein at most two of the groups Z, Y and X are N,
or anyone of its pharmaceutically acceptable salt,
for use as an agent for preventing, inhibiting or treating pathological or nonpathological conditions linked with premature aging.

Still in another particular variant, the compound of formula (I) may be defined as a compound of formula (IIa) as follows: 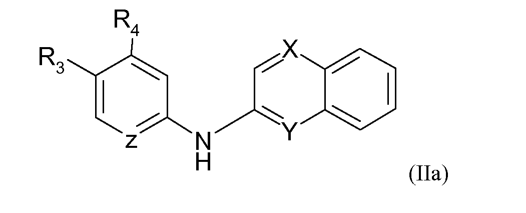 wherein:
Z is N or C, Y is N or C, X is N or C,
at least one of R₃ and R₄ is a hydrogen atom and the other is a -COOH group, a (C₁-C₃)alkyl group or a (C₁-C₃)fluoroalkoxy group, or anyone of its pharmaceutically acceptable salt.

Therefore, the present invention extends to a compound of formula (IIa) as defined above for use as an agent for preventing, inhibiting or treating pathological or nonpathological conditions linked with premature aging.

The present invention further relates to a compound of formula (IIb) 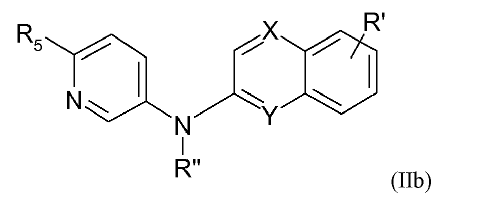 wherein:
Y is N or C,
X is N or C,
R₅ is a hydrogen atom, a halogen atom or a group chosen among a (C₁-C₃)alkyl group, a -CN group, a(C₁-C₃)alkoxy group, a -NO₂ group and a (C₁-C₃)fluoroalkyl group, and
R' and R" are as defined above,
or anyone of its pharmaceutically acceptable salt,
for use as an agent for preventing, inhibiting or treating pathological or nonpathological conditions linked with premature aging.

The present invention further relates to a compound of formula (IIc) 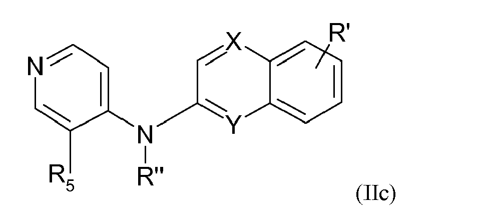 wherein:
Y is N or C,
X is N or C,
R₅ is a hydrogen atom, a halogen atom or a group chosen among a (C₁-C₃)alkyl group, a -CN group, a (C₁-C₃)alkoxy group, a -NO₂ group and a (C₁-C₃)fluoroalkyl group, and
R' and R" are as defined above,
or anyone of its pharmaceutically acceptable salt,
for use as an agent for preventing, inhibiting or treating pathological or nonpathological conditions linked with premature aging.

According to a first particular embodiment, an additional subject-matter of the present invention is a compound of formula (Ia) 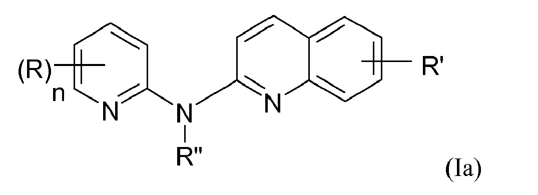 wherein:
R independently represent a hydrogen atom, a halogen atom, or a group chosen among a (C₁-C₃)alkyl group, a -CN group, a -COOH group, a (C₁-C₃)fluoroalkyl group, a (C1-C₃)fluoroalkoxy group, a -NO₂ group and a (C₁-C₃)alkoxy group,
R" is as defined above and is advantageously a hydrogen atom,
n is 1 or 2, and advantageously 1, and
R' is a hydrogen atom or a group chosen among a (C₁-C₃)alkyl group, a halogen atom, a hydroxy group, a -COOH group and a -CN group,
or one of its pharmaceutically acceptable salt,
for use as an agent for preventing, inhibiting or treating pathological or nonpathological conditions linked with premature aging.

The present invention further relates to a compound of formula (Ia) as defined above, as such,
wherein:
R, R" and n are as defined above,
R' is a hydrogen atom or a group chosen among a (C₁-C₃)alkyl group, a halogen atom, a -COOH group and a -CN group, and
wherein R and R' are not simultaneously a hydrogen atom or a methyl group and R is not a bromine atom,
or one of its pharmaceutically acceptable salt.

Still according to this particular embodiment, the present invention more particularly focuses on compounds of formula (Ia') 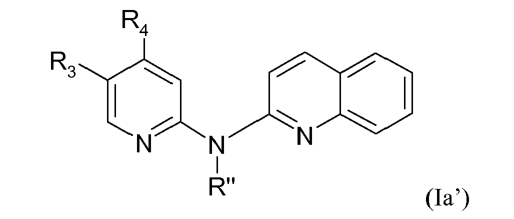 wherein:
at least one of R₃ and R₄ is a hydrogen atom and the other is a hydrogen atom, a -COOH group or a (C₁-C₃)alkyl group, and
R" is as defined above and is advantageously a hydrogen atom,
or one of its pharmaceutically acceptable salt,
for use as an agent for preventing, inhibiting or treating pathological or nonpathological conditions linked with premature aging.

According to a second particular embodiment, an additional subject-matter of the present invention is a compound of formula (Ib) 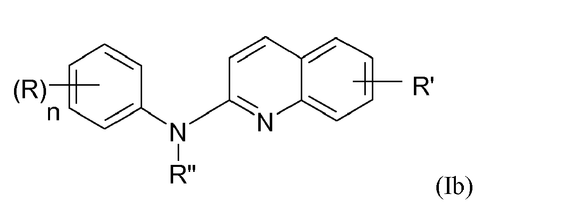 wherein:
R independently represent a hydrogen atom, a halogen atom, or a group chosen among a (C₁-C₃)alkyl group, a -CN group, a -COOH group, a (C₁-C₃)fluoroalkyl group, a (C₁-C₃)fluoroalkoxy group, a -NO₂ group, a -NR₁R₂ group and a (C₁-C₃)alkoxy group,
R₁ and R₂ are independently a hydrogen atom or a (C₁-C₃)alkyl group,
n is 1 or 2, and advantageously 1,
R' is a hydrogen atom or a group chosen among a (C₁-C₃)alkyl group, a halogen atom, a hydroxy group, a -COOH group and a -CN group, and
R" is as defined above and is advantageously a hydrogen atom,
or one of its pharmaceutically acceptable salt,
for use as an agent for preventing, inhibiting or treating pathological or nonpathological conditions linked with premature aging.

The present invention further relates to a compound of formula (Ib) as defined above, as such
wherein:
R' and R" are as defined above,
n is 1, and
R is a (C₁-C₃)fluoroalkoxy group,
or one of its pharmaceutically acceptable salt.

Still according to this particular embodiment, the present invention more particularly focuses on compounds of formula (Ib') 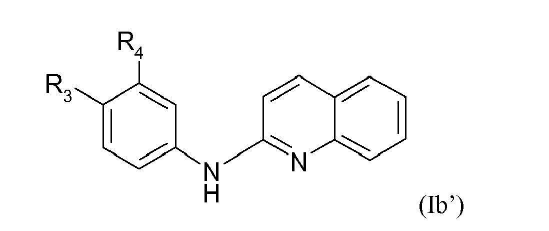 wherein:
at least one of R₃ and R₄ is a hydrogen atom and the other is a (C₁-C₃)fluoroalkoxy group or a (C₁-C₄)alkoxy group,
or one of its pharmaceutically acceptable salt,
for use as an agent for preventing, inhibiting or treating pathological or nonpathological conditions linked with premature aging.

According to a third particular embodiment, an additional subject-matter of the present invention is a compound of formula (Ic) 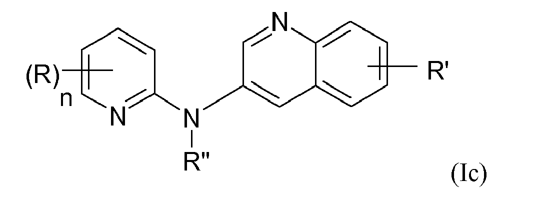 wherein:
R independently represent a hydrogen atom, a halogen atom, or a group chosen among a (C₁-C₃)alkyl group a -CN group, a -COOH group, a (C₁-C₃)fluoroalkyl group, a -NO₂ group and a (C₁-C₃)alkoxy group,
n is 1 or 2, and advantageously 1,
R' is a hydrogen atom or a (C₁-C₃)alkyl group, and in particular is a hydrogen atom, and
R" is as defined above and is advantageously a hydrogen atom,
or one of its pharmaceutically acceptable salt,
for use as an agent for preventing, inhibiting or treating pathological or nonpathological conditions linked with premature aging.

The present invention further relates to a compound of formula (Ic) as defined above, as such
wherein:
R, R' R" and n are as defined above, and
wherein R and R' are not simultaneously a hydrogen atom,
or one of its pharmaceutically acceptable salt.

Still according to this particular embodiment, the present invention more particularly focuses on compounds of formula (Ic'): 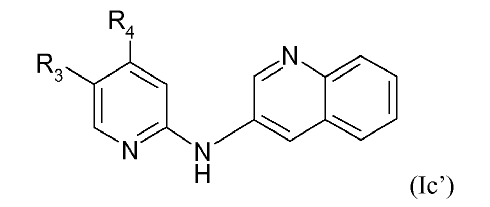 wherein:
at least one of R₃ and R₄ is a hydrogen atom and the other is a (C₁-C₃)alkyl group,
or one of its pharmaceutically acceptable salt,
for use as an agent for preventing, inhibiting or treating pathological or nonpathological conditions linked with premature aging.

According to a fourth particular embodiment, an additional subject-matter of the present invention is a compound of formula (Id): 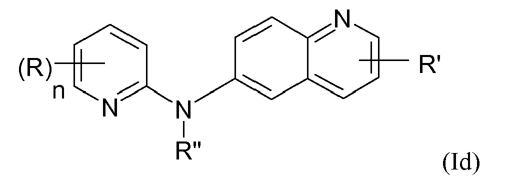 wherein:
R independently represent a hydrogen atom, a halogen atom, or a group chosen among a (C₁-C₃)alkyl group, a -CN group, a -COOH group, a (C₁-C₃)fluoroalkyl group, a (C₁-C₃)fluoroalkoxy group and a -NO₂ group,
n is 1 or 2, and advantageously 1,
R' is a hydrogen atom or a (C₁-C₃)alkyl group, and in particular is a hydrogen atom, and
R" is as defined above and is advantageously a hydrogen atom,
or one of its pharmaceutically acceptable salt,
for use as an agent for preventing, inhibiting or treating pathological or nonpathological conditions linked with premature aging.

The compound of formula (Id) as such and as defined above also form part of the present invention, with the proviso that when R' is a hydrogen atom, R is different from a -NO₂ group, or one of its pharmaceutically acceptable salt.

The compounds of formulae (I), (IIa), (IIb), (IIc), (Ia), (Ib), (Ic) and (Id) can comprise one or more asymmetric carbon atoms. They can thus exist in the form of enantiomers or of diastereoisomers. These enantiomers, diastereoisomers and their mixtures, including the racemic mixtures, are encompassed within the scope of the present invention.

According to a preferred embodiment of the present invention, the compound is chosen from:
- **(1)** (8-Chloro-quinolin-2-yl)-pyridin-2-yl-amine,
- **(2)** 2-(Quinolin-2-ylamino)-isonicotinic acid,
- **(3)** (4-Methyl-pyridin-2-yl)-quinolin-2-yl-amine,
- **(4)** Pyridin-2-yl-quinolin-2-yl-amine,
- **(5)** 2-(8-Chloro-quinolin-2-ylamino)-isonicotinic acid,
- **(6)** (8-Chloro-quinolin-2-yl)-(4-methyl-pyridin-2-yl)-amine,
- **(7)** 6-(Quinolin-2-ylamino)-nicotinonitrile,
- **(8)** Quinolin-2-yl-(4-trifluoromethoxy-phenyl)-amine,
- **(9)** Pyridin-2-yl-quinolin-3-yl-amine,
- **(10)** (3-Methoxy-pyridin-2-yl)-quinolin-3-yl-amine,
- **(11)** Quinolin-3-yl-(5-trifluoromethyl-pyridin-2-yl)-amine,
- **(12)** (5-Nitro-pyridin-2-yl)-quinolin-3-yl-amine,
- **(13)** (5-Methyl-pyridin-2-yl)-quinolin-3-yl-amine,
- **(14)** 2-(Quinolin-3-ylamino)-isonicotinic acid,
- **(15)** Quinolin-6-yl-(5-trifluoromethyl-pyridin-2-yl)-amine,
- **(16)** (6-Methyl-pyridin-2-yl)-quinolin-6-yl-amine, and
- their pharmaceutically acceptable salts.

Among said compounds, compounds (2), (3), (4) and (8) are of particular interest.

The present invention therefore extends to compounds (2), (3), (4) and (8) or one of its pharmaceutically acceptable salts for use as an agent for preventing, inhibiting or treating pathological or nonpathological conditions linked with premature aging.

Some compounds of said compounds (1) to (16), which are also reported in table I below, form part of the invention, as well as their pharmaceutically acceptable salts, such as hydrochloride, tartrate and fumarate: compounds (**1**), (**2**), (**5**), (**6**), (**7**), (**8**), (**10**), (**11**), (**12**), (**13**), (**14**), (**15**) and (**16**).

The compounds of the present invention can be prepared by conventional methods of organic synthesis practiced by those skilled in the art. The general reaction sequences outlined below represent a general method useful for preparing the compounds of the present invention and are not meant to be limiting in scope or utility.

The compounds of general formula (I) can be prepared according to scheme 1 below.

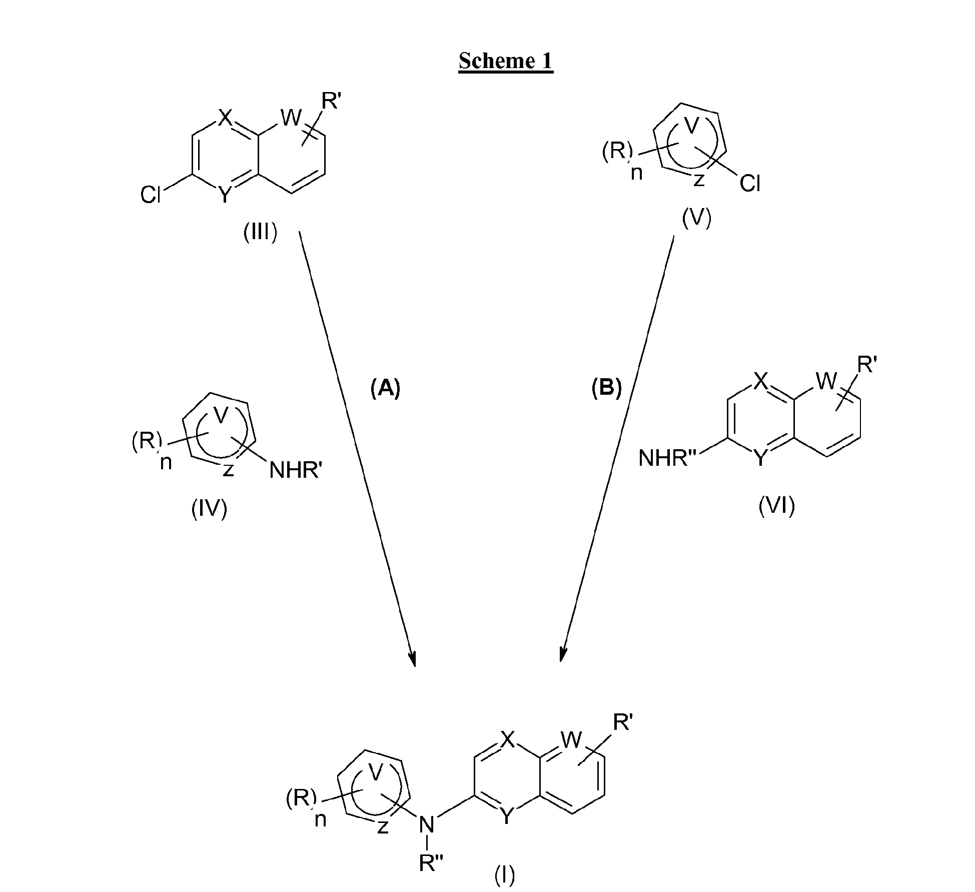

As appears in said scheme two routes are available for recovering a compound of formula (I) according to the present invention.

The synthesis is based on a coupling reaction alternatively starting from a chloro-bicyle of formula (III), wherein X, Y, W, R' and R" are as defined above or from a chloro-monocycle of formula (V), wherein Z, V, n and R are as defined above.

According to route (A), the compound of formula (III) is placed in a protic solvent such as *tert*-butanol. The compound of formula (IV) is then added in a molar ratio ranging from1 and 1.5 with respect to the compound of formula (III) in presence of an inorganic base, such as Cs₂CO₃ in a molar ratio ranging from 1 and 2, in the presence of a diphosphine, such as Xantphos (4,5-Bis(diphenylphosphino)-9,9-dimethylxanthene) in an amount ranging from 2 mol% and 10 mol% relative to the total amount of compound of formula (III), and in the presence of a catalyst, such as Pd(OA_{C})₂ in an amount ranging from 2 mol% and 10 mol% relative to the total amount of compound of formula (III). The reaction mixture can then be heated at a temperature ranging from 80 and 120°C, for example at 90°C and stirred for a time ranging form 15 and 25 hours, for example during 20 hours under inert gas and for example argon. The reaction mixture can be concentrated under reduced pressure.

According to route (B) the compound of formula (V) is placed in a protic solvent such as *tert*-butanol. The compound of formula (VI) is then added in a molar ratio ranging from1 and 1.5 with respect to the compound of formula (V) in presence of an inorganic base, such as Cs₂CO₃ in a molar ratio ranging from 1 and 2, in the presence of a diphosphine, such as Xantphos (4,5-Bis(diphenylphosphino)-9,9-dimethylxanthene) in an amount ranging from 2 mol% and 10 mol% relative to the total amount of compound of formula (V), and in the presence of a catalyst, such as Pd(OAc)₂ in an amount ranging from 2 mol% and 10 mol% relative to the total amount of compound of formula (V). The reaction mixture can then be heated at a temperature ranging from 80 and 120°C, for example at 90°C and stirred for a time ranging form 15 and 25 hours, for example during 20 hours under inert gas and for example argon. The reaction mixture can be concentrated under reduced pressure.

The starting compounds of formula (III), (IV), (V) and (VI) are commercially available or can be prepared according to methods known to the person skilled in the art.

The chemical structures and physical data of some compounds of formula (I) of the invention are illustrated in the following Table I.

**Table I**

| | | | |
|---|---|---|---|
| 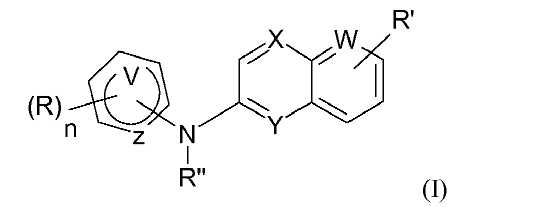 | | | |

| **N°** | 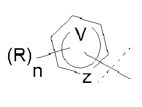 | 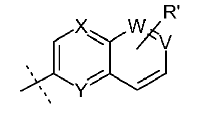 | **MS [M+H]⁺** |
|---|---|---|---|
| Formula (Ia) | | | |
| **1** | 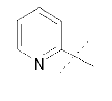 | 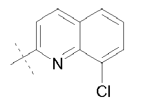 | 256.1 |
| **2** | 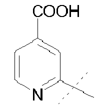 | 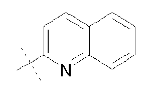 | 266.1 |
| **3** | 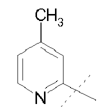 | 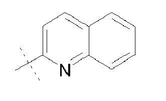 | 236.2 |
| **4** | 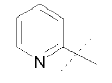 | 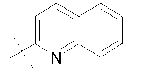 | 222.2 |
| **5** | 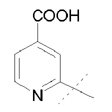 | 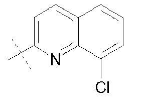 | 300.1 |
| **6** | 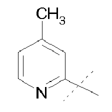 | 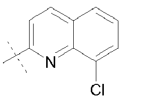 | 270.1 |
| **7** | 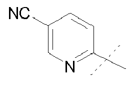 | 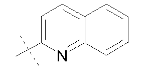 | 247.2 |
| Formula (Ib) | | | |
| **8** | 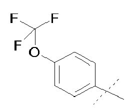 | 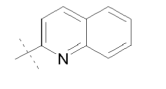 | 305.0 |
| Formula (Ic) | | | |
| **9** | 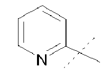 | 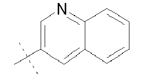 | 222.2 |
| **10** | 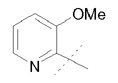 | 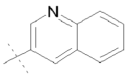 | 252.1 |
| **11** | 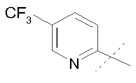 | 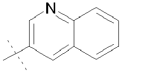 | 290.1 |
| **12** | 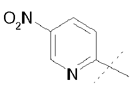 | 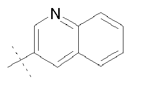 | 267.1 |
| **13** | 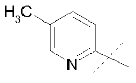 | 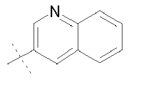 | 236.1 |
| **14** | 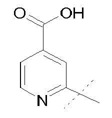 | 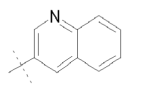 | 266.1 |
| Formula (Id) | | | |
| **15** | 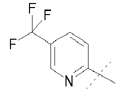 | 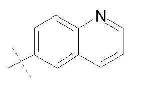 | 290.1 |
| **16** | 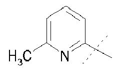 | 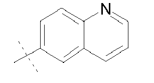 | 236.1 |

| | | | |
|---|---|---|---|
| MS: Mass Spectrometry ESI + | | | |

The following examples illustrate in detail the preparation of compounds (2), (3), (4) and (8) according to the invention. The structures of the products obtained have been confirmed by NMR spectra.

### EXAMPLES

### Typical procedure for Pd-catalysed aminations

To a solution of 2-chloro quinoline (82 mg, 0.5 mmol, 1 equiv) in *tert*-butanol (2 mL) were added the amino pyridine derivative / aniline (0.55 mmol, 1.1 equiv), Cs₂CO₃ (456 mg, 1.4 mmol, 2.8 equiv), Xantphos (5.8 mg, 0.01 mmol, 2 mol%), Pd(OAc)₂ (2.2 mg, 0.01 mmol, 2 mol%). The reaction mixture was heated at 90°C and stirred for 20 hours under argon. The reaction mixture was concentrated under reduced pressure. The residue was purified by column chromatography on silica gel to yield compounds (2), (3), (4) and (8).

### Example 1: 2-(Quinolin-2-ylamino)-isonicotinic acid - (2) of table 1

¹H NMR (300 MHz, DMSO) δ 13.16 (s, 1H), 8.72 (d, J = 5.2, 1H), 8.63 (d, J = 9.0, 1H), 8.28 - 8.13 (m, 2H), 8.05 (d, J = 8.0, 1H), 7.90 (t, J = 7.5, 1H), 7.74 - 7.67 (m, 2H), 7.67 - 7.59 (m, 2H).

MS (electrospray) m/z (%) 266.1 (100) [M+H]⁺.

### Example 2: (4-Methyl-pyridin-2-yl)-quinolin-2-yl-amine - (3) of table 1

¹H NMR (300 MHz, CDC13) δ 8.92 (s, 1H), 8.21 (d, *J* = 5.3, 2H), 7.95 (d, *J* = 8.9, 1H), 7.89 (d, *J* = 8.4, 1H), 7.67 (d, *J* = 8.0, 1H), 7.62 (t, *J* = 7.7, 1H), 7.40 - 7.28 (m, 2H), 6.78 (d, *J* = 5.1, 1H), 2.41 (s, 3H).

¹³C NMR (75 MHz, CDC13) δ 154.3, 153.3, 149.5, 147.3, 137.7, 129.8, 127.6, 127.1, 124.6, 123.7, 118.7, 114.1, 113.4, 21.7.

MS (electrospray) m/z (%) 236.2 (100) [M+H]⁺.

### Example 3: Pyridin-2-yl-quinolin-2-yl-amine - (4) of table 1

¹H NMR (300 MHz, CDC13) δ 8.38 (d, *J* = 8.4, 1H), 8.31 (dd, *J* = 1.0,4.9, 1H), 8.01 (d, *J* = 8.9, 1H), 7.87 (d, *J* = 8.4, 1H), 7.77 - 7.68 (m, 3H), 7.64 (t, *J* = 7.7, 1H), 7.36 (t, *J* = 7.5, 1H), 7.31 (d, *J* = 8.9, 1H), 6.94 (dd, *J* = 5.0, 7.2, 1H).

¹³C NMR (75 MHz, CDC13) δ 154.1, 153.1, 147.8, 147.3, 138.3, 137.8, 129.9, 127.6, 127.2, 124.6, 123.8, 117.4, 114.0, 113.0.

MS (electrospray) m/z (%) 222.2 (100) [M+H]⁺.

### Example 4: Quinolin-2-yl-(4-trifluoromethoxy-phenyl)-amine - (8) of table 1

¹H NMR (300 MHz, CDC13) δ 7.97 (d, *J* = 8.8, 1H), 7.82 (d, *J* = 8.4, 1H), 7.69 (t, *J* = 9.4, 3H), 7.62 (t, *J* = 7.7, 1H), 7.34 (t, *J* = 7.5, 1H), 7.23 (d, *J* = 8.7, 2H), 6.92 (d, *J* = 8.9, 1H), 6.74 (s, 1H).

¹³C NMR (75 MHz, CDC13) δ 153.9, 147.6, 144.4, 139.3, 138.1, 130.1, 127.7, 127.1, 124.4, 123.7, 122.5, 122.2, 121.0, 119.1, 112.2.

MS (electrospray) m/z (%) 305.0 (100) [M+H]⁺.

### Example 5: Pharmacological data

The compounds of the invention have been the subject of pharmacological tests which have demonstrated their relevance as active substances in therapy and in particular for preventing, inhibiting or treating pathological or nonpathological conditions linked with premature aging.

The following materials and methods have been used.

### MATERIAL AND METHODS

### Minigene constructs reproduce aberrant splicing of LMNA mRNA, leading to HGPS

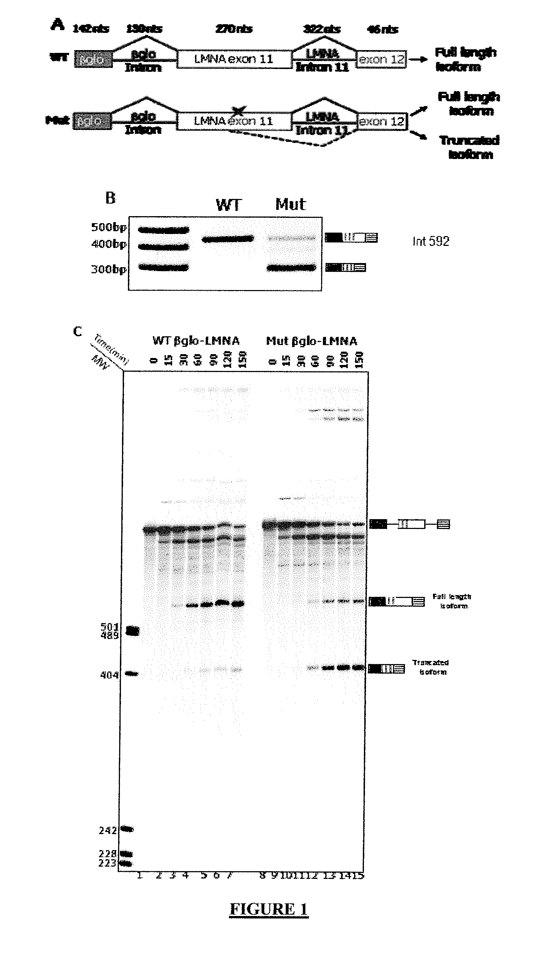

In order to identify and characterize the factor(s) involved in the use of the cryptic 5' splice site in exon 11 of LMNA, an *ex vivo* system has been developed that recapitulates this splicing event. The cloning of mutant and wild type constructs (Fig. 1A, for schematic representation) were carried out using a TOPO-TA cloning vector in which is inserted a minigene containing 142 nts of β-Globin fist exon, 130 nts β-Globin first intron, 270 nts LMNA exon 11 either wild type or mutant, 322 nts intron 11 and 46 nts exon 12. Using this system the splicing event activated by the GGC>GGT mutation in exon 11 of the LMNA gene was confirmed by transfections in cultured HeLa cells (Fig. 1B, lanes WT and Mut) as well as *in vitro* splicing experiments using in vitro synthesized radiolabeled substrate (Panel C). Transfection experiments of minigene constructs containing or not the point mutation demonstrated that like in Progeria patients the mutation leads to a switch from the use of the normal splice site (intron 11 position 1) to the use of the cryptic splice site upstream of the mutation (exon 11 position 1819) (Fig. 1, Panel C, compare lanes WT and Mut). Note that following a kinetics of *in vitro* splicing for 150 minutes, aberrant splicing is observed with the wild type substrate (Fig1C, Lanes 1-7), implying that the mutation is not a perquisite for cryptic splice site usage. The mutation simply enhances the efficacy of selection of this cryptic splice site (Fig1C, Lanes 8-15).

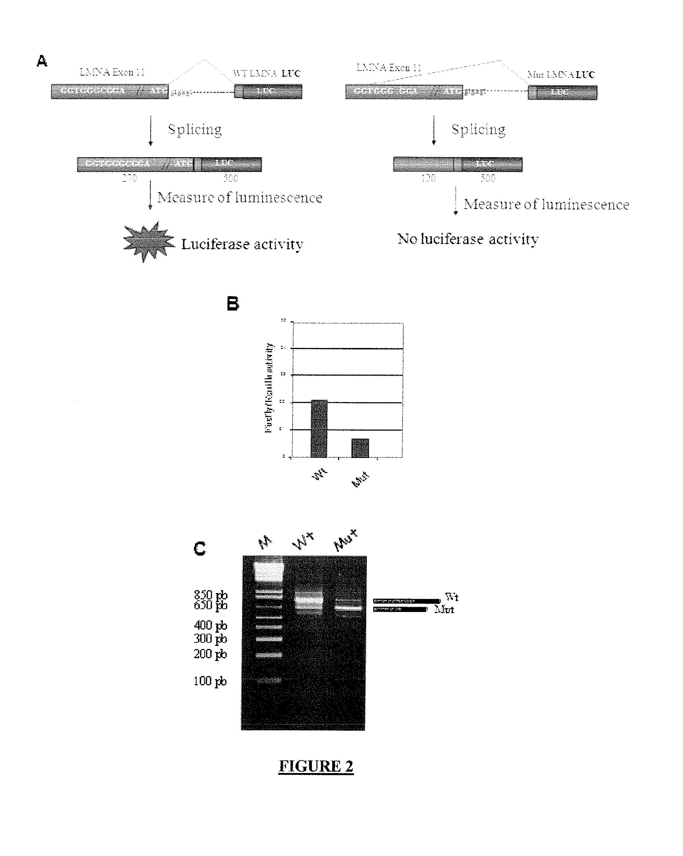

Advantage has been taken of the luciferase system. The luciferase assay is an extremely sensitive and rapid assay. Linear results are seen over at least eight orders of magnitude of enzyme concentration. Moreover, the luciferase assay is well suited for high-throughput applications. To conduct a Mid-throughput screening (MTS) for compounds repressing LMNA aberrant splicing, we have constructed a plasmid in which exon 11, intron 11 and part of exon 12 of LMNA gene were fused with luciferase cDNA (Fig. 2). Both wild type (WtLMNA-luc) and mutant (MutLMNA-luc) substrate harbouring exon 11 mutation have been constructed. In these constructs we have generated a single initiation codon in exon 11 such as correct splicing will lead to luciferase expression, while aberrant splicing will skip the initiation codon and thereby prevent luciferase expression. After transfection in HeLa cells, Luciferase assays (Fig. 2B) and RT-PCR (Fig. 2C) indicate that WtLMNA-luc produces predominantly wild type splicing and large amount of luciferase activity, whereas MutLMNA-luc recapitulate the aberrant splicing profile with reduced luciferase expression (Fig.2 B and C, compare Wt and Mut). In order to use this system in MTS, we have generated a stable 293 cell lines containing a single integrated copy of luciferase reporter containing LMNA mutation (MutLMNA-luc cell line) using the flp system from INVITROGEN. This system allows us to perform a MTS for compounds able to enhance luciferase activity.

***Plasmids constructs**.* LMNA sequences (1278 bp of exon 11, intron 11 and 46 bp of exon 11) were PCR-amplified from either control or patient's cells genomic DNA with specific primer PCR fragments were purified with Concert Rapid PCR purification system (Invitrogen) and subcloned at the BamHI and EcoRI restriction sites of the pSpβm3S1 plasmid containing the βGlobin cassette (Labourier et al., 1999 - Recognition of exonic splicing enhancer sequences by the Drosophila splicing repressor RSF1. Nucleic Acids Res. 27, 2377-2386) to give the βGlo3S1LMNAwt and βGlo3S1LMNAmut constructs. The chimeric βGlo-LMNA sequences were then inserted into the pcDNA3.1D/V5-His-TOPO vector (Invitrogen) to be used in transfection and in vitro splicing experiments. A single initiation codon ATG was kept in exon 11 of LMNA and LMNA sequences described above were fused at their 3' end to Fyrefly luciferase cDNA (LMNAlucWT) in order that removal of intron 11 generates a transcript that encode a fusion protein harbouring luciferase activity, whereas usage of the cryptic splice site of mutated exon 11 (LMNAIucMut) will remove the initiation codon preventing luciferase expression. Both sequences were cloned in pcDNA3 Flp-In vector (Invitrogen).

***Transfection and RT-PCR.*** HeLa cells transfections with splicing reporter constructs were performed with lipofectAMINE 2000 reagent (Invitrogen) according to the manufacturer's instructions. Twenty four hours after transfection, total RNA was purified with RNA-PLUSTM (Quantum Bioprobe). First strand cDNA was synthesized from 2 µg of RNA with the Amersham-Pharmacia First strand cDNA synthesis kit. For PCR analyses, 1/15 of the reaction was amplified with Taq polymerase (Invitrogen). The cycle number was kept to a minimum to maintain linearity. PCR products were separated on a 1.5% agarose gel containing ethidium bromide and visualized under UV light.

A stable 293 cell line containing a single copy of LMNAlucMut minigene was obtained using the Flp-In system from (Invitrogen) according to manufacture procedure. Several clones were obtained and only one clone was used to screen the whole chemical library (293FLP LMNA LUC cells #8).

***Nuclear extracts preparation, splicing and complementation assays.*** HeLa cells nuclear extracts were prepared according to (Dignam et al., 1983 - Eukaryotic gene transcription with purified components. Methods Enzymol. 101, 582-598). Pre-mRNA were synthesized by in vitro transcription in the presence of 20 units of T7 RNA polymerase, 1µg of the suitable linearized plasmids and 5 µM [α-³²P] UTP (3000 Ci/mmol) in 25 µl reactions according to manufacturer conditions. In vitro transcripts were quantified by Cerenkov counting. Splicing reactions were performed under standard conditions as described previously (Tazi et al., 1986 - A protein that specifically recognizes the 3' splice site of mammalian pre-mRNA introns is associated with a small nuclear ribonucleoprotein. Cell 47, 755-766). Splicing products were analyzed by electrophoresis on 7% denaturing polyacrylamide gels and revealed by autoradiography.

### Material

293FLP LMNA LUC cells #8

Hygromycin B at 50 mg/mL *(invitrogen 10687-010)*

Dulbecco's Modified Eagle Medium (D-MEM) (1X) + GlutaMAX, liquid *(invitrogen 31966-021)*

Dulbecco's Phosphate Buffered Saline (D-PBS) (1X), liquid *(invitrogen 14190-169)*

Trypsin 2.5%

Foetal calf serum (FCS)

Penicillin (P)

Streptomycin (S)

Passive Lysis Buffer (PLB) (5X) *(Promega)*

Bradford Reagent (B6916)

Luciferase assay buffer

96 Well Plate sterile, V-shape *(greiner bio-one 651180)*

96 Well Microplate sterile, flat bottom *(greiner bio-one 655180)*

96 Well Microplate, flat bottom, Chimney Well *(greiner bio-one 655075)*

CellTiter 96^{®} AQᵤₑₒᵤₛ One Solution *(Promega G3581)*

### Methods

### FIRST DAY

### Plate at 500 µM

In a 96 Well Plate sterile, V-shape one put 0.5 µl of drug compounds at 50 mM and then add 49,5 µl of 10% DMSO.

### Replica plate

One pipets 47 µl of drug at 500 µM and add 200 µL of DMEM + Hygromycin B. At this stage the concentration of drug compound is 10 µM. One shares out 100 µl in a 96 Well Microplate sterile, flat bottom (further called luciferase plate) and 50 µl in other one (further called toxicity plate).

One wash 293FLP LMNA LUC cells once with D-PBS then add 1 ml trypsin EDTA. Incubation at 37°C for 2-3 minutes is proceeded. Then one add 9 ml DMEM (with 10% FCS, P/S).

One takes 7 µl of cell suspension and adds 14 µL blue trypan to count cells. Meanwhile cell suspension is centrifugated at 1200 rpm for 5 minutes at room temperature (RT).

Cell concentration is brought at 105 cells per ml with DMEM + Hygromycin B to have 104 cells per 100 µl.

### Luciferase plate

100 µl of suspension cells is added (at 104 cells per 100 µl) so final concentration of compounds is 5 µM.

### Toxicity plate

50 µl of suspension cells is added. The final concentration of compounds is 5 µM.

### 48 HOURS LATER

### Toxicity plate

20 µl of CellTiter 96^{®} AQueous One Solution is added per well. Incubation is proceeded at 37°C for 2 h. Absorbance is red at 490 nm.

### Luciferase plate

Medium of the wells is gently removed then washed once by adding slowly 150 µl of D-PBS 1X. D-PBS is removed. 40 µl of PLB 1X is added and incubated at RT for 30 minutes.

20 µl of cell lysate is put in a 96 Well Microplate, flat bottom, Chimney Well. 70 µl of luciferin assay substrate is added. One read luminescence for 1 second.

200 µl of Bradford reagent is added on the remaining cell lysate (20 µl). Incubation is proceeded at RT for 30 min then one can read absorbance at 595 nm. A range has to be made. Usually 5 different concentrations are tested: 0.25, 0.5, 0.75, 1 and 1.25 mg/ml.

### RESULTS

The compounds according to the present invention demonstrate an increase of luciferase activity ranging between 3 and 7 fold compared to control untreated MutLMNA-luc cell line.

Therefore, the result of the tests carried out on the compounds disclosed in the present invention show that said compounds may be useful to inhibit, prevent and/or treat diseases with premature aging and that are likely related to an aberrant splicing of the nuclear lamin A gene. Among all, said disease may include Hutchinson Guilford Progeria Syndrome (HGPS), progeria, premature aging associated with HIV infection, muscular dystrophy, Charcot-Marie-Tooth disorder, Werner syndrome, but also atherosclerosis, insulin resistant type II diabetes, cataracts, osteoporosis and aging of the skin such as restrictive dermopathy.

For this purpose an effective amount of a said compound may be administered to a patient suffering from premature aging and in particular from progeria, and from the previous cited diseases.

The present invention is also related to the use of a compound of anyone of formula (I), (IIa), (IIb), (IIc), (Ia), (Ib), (Ic), (Id), and (1) to (16) or one of its pharmaceutically acceptable salts according to the present invention for the manufacture of a pharmaceutical composition intended for the treatment of pathological or nonpathological conditions linked with premature aging and in particular progeria.

The present invention also encompasses pharmaceutical compositions comprising at least a compound chosen among new compounds of formula (Ia), (Ib), (Ic) or (Id) as defined above and compounds (1) to (16), as defined above or any pharmaceutically acceptable salt thereof.

Thus, these pharmaceutical compositions contain an effective amount of said compound, and one or more pharmaceutical excipients.

The aforementioned excipients are selected according to the dosage form and the desired mode of administration.

In this context they can be present in any pharmaceutical form which is suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions, in doses which enable the daily administration of from 0.1 to 1000 mg of active substance.

The present invention is also related to the use of a compound of anyone of formula (I), (IIa), (IIb), (IIc), (Ia), (Ib), (Ic), (Id) and (1) to (16) or one of its pharmaceutically acceptable salts according to the present invention for the manufacture of a pharmaceutical composition intended for inhibiting, preventing and/or treating pathological or nonpathological conditions linked with premature aging and in particular progeria but also all the previous listed diseases.

The present invention further relates to a method of treatment of patients suffering form premature aging or anyone of the previous listed disease, which comprises at least a step of administration to a patient suffering thereof of an effective amount of a compound of anyone of formula (I), (IIa), (IIb), (IIc), (Ia), (Ib), (Ic), (Id) and (1) to (16) or one of its pharmaceutically acceptable salts.

## Claims

1. A compound of formula (I): 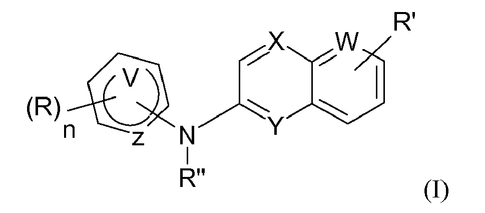 wherein: 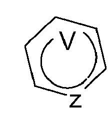
means an aromatic ring wherein V is C or N and when V is N, V is in ortho, meta or para of Z, i.e. forms respectively a pyridazine, a pyrimidine or a pyrazine group,
R independently represents a hydrogen atom, a halogen atom or a group chosen among a (C₁-C₃)alkyl group, a -CN group, a -COOH group, a (C₁-C₃)fluoroalkyl group, a (C₁-C₃)fluoroalkoxy group, a -NO₂ group, a -NR₁R₂ group and a (C₁-C₃)alkoxy group,
R₁ and R₂ are independently a hydrogen atom or a (C₁-C₃)alkyl group,
n is 1 or 2,
R' is a hydrogen atom or a group chosen among a (C₁-C₃)alkyl group, a halogen atom, a hydroxy group, a -COOH group and a -CN group,
R" is a hydrogen atom or a (C₁-C₄)alkyl group,
Z is N or C,
Y is N or C,
X is NorC,
W is N or C,
and wherein at most two of the groups Z, Y, X and W are N,
or anyone of its pharmaceutically acceptable salt,
for use as an agent for preventing, inhibiting or treating pathological or nonpathological conditions linked with premature aging.

2. A compound of formula (I) according to claim 1, wherein alternatively:
- Z is N, V is C, Y is N, X is C and W is C,
- Z is C, V is C, Y is N, X is C and W is C,
- Z is N, V is C, Y is C, X is N and W is C,
- Z is N, V is C, Y is C, X is C and W is N,
for use as an agent for preventing, inhibiting or treating pathological or nonpathological conditions linked with premature aging.

3. A compound of formula (I) according to claim 1 or 2, wherein
Z is N or C, V is C, Y is N or C, X is N or C and W is C,
n is equal to 1,
R is a hydrogen atom, a -COOH group, a (C₁-C₃)alkyl group or a (C₁-C₃)fluoroalkoxy group,
R' is a hydrogen atom,
R" is a hydrogen atom, and
wherein at most two of the groups Z, Y and X are N,
for use as an agent for preventing, inhibiting or treating pathological or nonpathological conditions linked with premature aging.

4. A compound of formula (I) according to anyone of the preceding claims, defined by a formula (IIa) 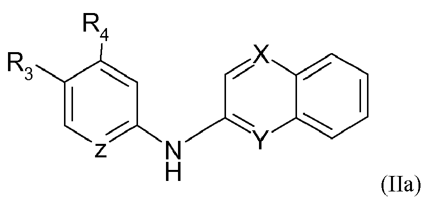 wherein:
Z is N or C, Y is N or C, X is N or C,
at least one of R₃ and R₄ is a hydrogen atom and the other is a -COOH group, a (C₁-C₃)alkyl group or a (C₁-C₃)fluoroalkoxy group,
or anyone of its pharmaceutically acceptable salt,
for use as an agent for preventing, inhibiting or treating pathological or nonpathological conditions linked with premature aging.

5. A compound of formula (Ia) according to anyone of the preceding claims 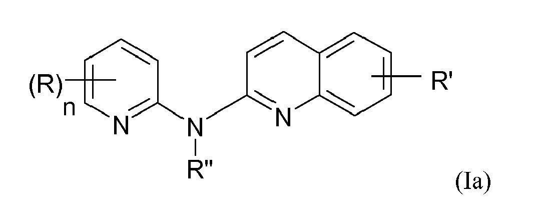 wherein:
R independently represent a hydrogen atom, a halogen atom or a group chosen among a (C₁-C₃)alkyl group, a -CN group, a -COOH group, a (C₁-C₃)fluoroalkyl group, a (C₁-C₃)fluoroalkoxy group, a -NO₂ group, a (C₁-C₃)alkoxy group,
R" is as defined in claim 1 and is advantageously a hydrogen atom,
n is 1 or 2, and advantageously 1,
R' is a hydrogen atom or a group chosen among a (C₁-C₃)alkyl group, a halogen atom, a hydroxy group, a -COOH group and a -CN group,
or one of its pharmaceutically acceptable salt,
for use as an agent for preventing, inhibiting or treating pathological or nonpathological conditions linked with premature aging.

6. A compound of formula (Ia), which formula (Ia) is such as defined in the preceding claim,
wherein
R, R" and n are as defined in the preceding claim,
R' is a hydrogen atom or a group chosen among a (C₁-C₃)alkyl group, a halogen atom, a -COOH group and a -CN group, and
wherein R and R' are not simultaneously a hydrogen atom or a methyl group and R is not a bromine atom,
or one of its pharmaceutically acceptable salt.

7. A compound of formula (Ib) according to anyone of claims 1 to 4 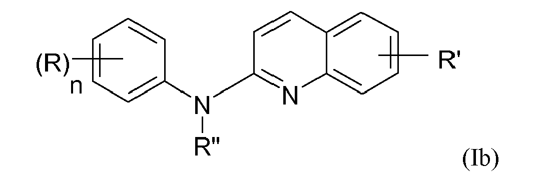 wherein:
R independently represents a hydrogen atom, a halogen atom or a group chosen among a (C₁-C₃)alkyl group, a -CN group, a -COOH group, a (C₁-C₃)fluoroalkyl group, a (C₁-C₃)fluoroalkoxy group, a -NO₂ group, a -NR₁R₂ group and a (C₁-C₃)alkoxy group,
R₁ and R₂ are independently a hydrogen atom or a (C₁-C₃)alkyl group,
n is 1 or 2, and advantageously 1,
R' is a hydrogen atom or a group chosen among a (C₁-C₃)alkyl group, a halogen atom, a hydroxy group, a -COOH group and a -CN group, and
R" is as defined in claim 1 and is advantageously a hydrogen atom,
or one of its pharmaceutically acceptable salt,
for use as an agent for preventing, inhibiting or treating pathological or nonpathological conditions linked with premature aging.

8. A compound of formula (Ib), which formula (Ib) is such as defined in the preceding claim, wherein:
R' and R" are as defined in the preceding claim,
n is 1, and
R is a (C₁-C₃)fluoroalkoxy group,
or one of its pharmaceutically acceptable salt.

9. A compound of formula (Ic) according to anyone of claims 1 to 4: 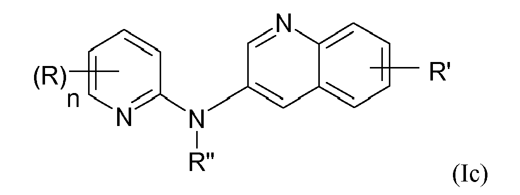 wherein:
R independently represent a hydrogen atom, a halogen atom, or a group chosen among a (C₁-C₃)alkyl group, a -CN group, a -COOH group, a (C₁-C₃)fluoroalkyl group, a -NO₂ group, and a (C₁-C₃)alkoxy group,
n is 1 or 2, and advantageously 1,
R' is a hydrogen atom or a (C₁-C₃)alkyl group, and in particular is a hydrogen atom, and
R" is as defined in the preceding claim and is advantageously a hydrogen atom,
or one of its pharmaceutically acceptable salt,
for use as an agent for preventing, inhibiting or treating pathological or nonpathological conditions linked with premature aging.

10. A compound of formula (Ic), which formula (Ic) is such as defined in the preceding claim, wherein:
R, R' R" and n are as defined in the preceding claim, and
wherein R and R' are not simultaneously a hydrogen atom,
or one of its pharmaceutically acceptable salt.

11. A compound of formula (Id) according to anyone of the claims 1 to 4 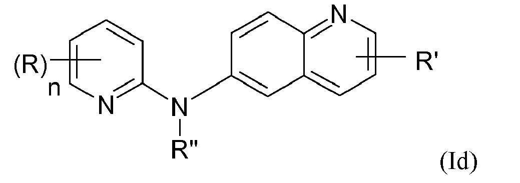 wherein:
R independently represent a hydrogen atom, a halogen atom or a group chosen among a (C₁-C₃)alkyl group, a -CN group, a -COOH group, a (C₁-C₃)fluoroalkyl group, a (C₁-C₃)fluoroalkoxy group and a -NO₂ group,
n is 1 or 2, and advantageously 1,
R' is a hydrogen atom or a (C₁-C₃)alkyl group, and in particular is a hydrogen atom, and
R" is as defined in claim 1 and is advantageously a hydrogen atom,
or one of its pharmaceutically acceptable salt,
for use as an agent for preventing, inhibiting or treating pathological or nonpathological conditions linked with premature aging.

12. A compound of formula (Id), which formula (Id) is such as defined in the preceding claim, with the proviso that when R' is a hydrogen atom, R is different from a -NO₂ group,
or one of its pharmaceutically acceptable salt.

13. A compound chosen among:
- **(1)** (8-Chloro-quinolin-2-yl)-pyridin-2-yl-amine,
- **(2)** 2-(Quinolin-2-ylamino)-isonicotinic acid,
- **(3)** (4-Methyl-pyridin-2-yl)-quinolin-2-yl-amine,
- **(4)** Pyridin-2-yl-quinolin-2-yl-amine,
- **(5)** 2-(8-Chloro-quinolin-2-ylamino)-isonicotinic acid,
- **(6)** (8-Chloro-quinolin-2-yl)-(4-methyl-pyridin-2-yl)-amine,
- **(7)** 6-(Quinolin-2-ylamino)-nicotinonitrile,
- **(8)** Quinolin-2-yl-(4-trifluoromethoxy-phenyl)-amine,
- **(9)** Pyridin-2-yl-quinolin-3-yl-amine,
- **(10)** (3-Methoxy-pyridin-2-yl)-quinolin-3-yl-amine,
- **(11)** Quinolin-3-yl-(5-trifluoromethyl-pyridin-2-yl)-amine,
- **(12)** (5-Nitro-pyridin-2-yl)-quinolin-3-yl-amine,
- **(13)** (5-Methyl-pyridin-2-yl)-quinolin-3-yl-amine,
- **(14)** 2-(Quinolin-3-ylamino)-isonicotinic acid,
- **(15)** Quinolin-6-yl-(5-trifluoromethyl-pyridin-2-yl)-amine,
- **(16)** (6-Methyl-pyridin-2-yl)-quinolin-6-yl-amine,
- their pharmaceutically acceptable salts
for use as an agent for preventing, inhibiting or treating pathological or nonpathological conditions linked with premature aging.

14. A compound chosen among the compounds **(1), (2), (5), (6), (7), (8), (10), (11), (12),** and **(13), (14), (15)** and **(16)** as well as their pharmaceutically acceptable salts, such as hydrochloride, tartrate and fumarate.

15. A pharmaceutical composition comprising at least one compound as defined in anyone of claims 6, 8, 10 12 and 14.
